# EUROPEAN PATENT APPLICATION

(11) **EP 1 252 894 A1**
(43) Date of publication of application: **30.10.2002**
(21) Application number: 01401077.1
(22) Date of filing: 26.04.2001
(51) Int. Cl.: A61K 38/16, A61K 38/08, C12N 15/85, A61K 48/00, A61P 17/06, A61P 27/02, A61P 35/00

(54) **Use of agt and its derivatives for manufacturing anti-angiogenesis pharmaceutical compositions**

(71) Applicant: INSTITUT NATIONAL DE LA SANTE ET DE LA RECHERCHE MEDICALE (INSERM), 75654 Paris Cédex 13 (FR)
(72) Inventor: Celerier, Jerome, 92260 Fontenay aux Roses (FR); Cruz, Amauri, 75005 Paris (FR); Lamande, Noel, 94600 Choisy le Roi (FR); Gasc, Jean Marie, 75005 Paris (FR); Corvol, Pierre, 75007 Paris (FR)
(74) Representative: Michelet, Alain

(57) **Abstract**

An object of the present invention is the use of at least one inhibitor of angiogenesis consisting of AGT or derivatives thereof in the manufacture of a drug for treating pathologies or disorders notably angiogenesis-dependent or mediated pathologies generally characterised by excessive angiogenesis.

The present invention also relates to a cell transfected with at least one expression vector wherein said expression vector hosts a recombinant nucleic acid encoding an AGT or one of its derivatives for use in therapy of angiogenesis-mediated pathologies.

The present invention further relates to an expression vector for use in said cell.

## Description

The present invention relates in general to the use of at least one inhibitor of angiogenesis in the manufacture of a drug for treating pathologies or disorders generally characterised by excessive angiogenesis, in particular ischemic retinopathies such as proliferative diabetic retinopathy and age-related macular degeneration, arthritis, cancer, psoriasis, cancer and metastasis, psoriasis, arthritis, duodenal ulcers, infantile hemangiomas, disorders of female reproduction associated with angiogenesis dysfunction.

More particularly the present invention pertains to the use of molecules belonging to the subfamily of non-inhibitory serpins.

Serpins appeared to have unexpected functions in angiogenesis. In general angiogenesis refers to the growth of new blood vessels from pre-existing ones. The properties of two anti-angiogenic serpins have been identified by their ability to block endothelial cell growth in culture. Indeed, prior art documents referenced in the present invention 18, 26 teach that the reactive center loop (RCL)-cleaved form of anti-thrombin III (ATIII), a protein well known for its anti-clotting activity, have anti-angiogenic properties.

Moreover, it is known from document herein referenced 7 that one other serpin, PEDF, which is a protein with neurotropic activity, is a potent inhibitor of angiogenesis, The anti-angiogenic activity of PEDF was discovered from a systematic search of putative anti-angiogenic factors present in retinoblastoma conditioned culture media which was able to prevent vessels from invading the cornea and vitreous. PEDF is controlled by oxygen concentration and appears to physiologically regulate angiogenesis in the eye during development. Altogether, these results suggest that PEDF may be of therapeutic use, especially in retinopathies where pathological neovascularisation compromises vision and leads to blindness.

In addition, published document, ref. 39, teaches that the purified serpin named maspin effectively inhibited neovascularisation *in vivo.* Furthermore, maspin possess an antitumor activity, which may also act by inhibiting the angiogenesis.

Altogether, these studies suggest a common mechanism for inhibition by serpins of proliferation and/or migration of endothelial cells during angiogenesis. The mechanism is clearly not related to an inhibitory serpin function since PEDF and maspin are non-inhibitory serpins and because the RCL-cleaved ATIII has lost the capability to bind its specific target. The tertiary structure of the cleaved form of ATIII was among the first serpin structures to be determined, and for maspin a serpin fold has been predicted. Therefore, the anti-angiogenic effect of serpins may pertain to a structural feature common to serpins rather than to their activity.

Among the serpin subfamily, angiotensinogen (AGT) is the precursor of angiotensin I (Angl), an inactive decapeptide which is converted into angiotensin II (Ang II), the main effector of the renin-angiotensin system (RAS).

According to the present inventors, the only role known so far of AGT is to be the substrate of the highly specific aspartyl protease, renin. Renin cleaves the N-terminal end of AGT to generate Angl, leaving intact a much larger fragment (97.8% of the whole amino acid sequence), which until now did not have any known function, called des(Angl)angiotensinogen (des(Angl)AGT).

AGT biochemical, enzymological and structural characterisations have been thoroughly investigated because it is the rate limiting step in the first reaction of the RAS cascade: its plasma concentration (1 µM) is close to its affinity (Km) for renin. The liver is the main site of AGT synthesis but other sites of production have been well documented such as glial cells, adipocytes, kidney, and the wall of large vessels, such as aorta. Plasma AGT concentration is regulated by hormonal factors (estrogens, glucocorticoids, thyroid hormone, angiotensin II) and angiotensin converting enzyme inhibitor treatment. Plasma AGT concentration is also dependent on AGT genotype. An AGT gene variant at position 235 (235T) is associated with a 10-20% increase in plasma AGT concentration and with high blood pressure.

Des(Angl)AGT, being devoided of any conspicuous role, was considered as a degradation product and thus was not extensively studied. It has been suggested that des(Angl)AGT may inhibit the renin AGT reaction, but the present inventors were unable to confirm this observation by using pure renin and pure des(Ang I)AGT (Corvol et al., unpublished data). AGT shares aminoacid sequence and structural homologies with the serine protease inhibitor (serpin) family of proteins although it has no inhibitor activity, like ovalbumin, pigment epithelial derived factor (PEDF) and maspin.

Human AGT has been modelised as a serpin by Streatfeild-James, 1998 and by the present inventors. The N-terminal moiety, which bears the decapeptide angiotensin I, is excluded from the model of the present invention. The rest of the molecule, which represent 97,8% of the whole aminoacid sequence, is named des(Angl)AGT. AGT and des(Angl)AGT belong to the class of non-inhibitory serpins and have been predicted to have a serpin fold structure. But nevertheless, 85% of the primary sequence AGT or des(Angl)AGT allows a prediction of folding like a serpin.

AGT is, indeed, unable to undergo the classical stressed-relaxed pathway of the inhibitory serpins such as anti-thrombin III. In addition, AGT can be cleaved by the staphylococcus V₈ proteinase in its reactive center-cleaved AGT form herein abbreviated "RCL-cleaved AGT".

In view of the prior art described above, according to the present inventors there are problem(s) remaining to be solved notably as follows:
1. treat the above mentioned pathologies without or with less secondary or side effects.
2. to the best knowledge of the present inventors there are no molecule or therapeutic strategies which are currently available in human therapeutic field.
   - in addition, treatments currently proposed for angiogenesis-dependent / mediated diseases are not devoid of side effects.
   - since more than one anti-angiogenic pathway exist, it is possible that a new inhibitor or a combination of two or more types of inhibitors could be more effective. It is thus necessary to discover new inhibitors of angiogenesis to find more appropriate treatments.

Therefore, according to the present inventors there is still a need to improve efficacy and diminish toxicity in the treatment of angiogenesis-dependent / mediated diseases.

One of the aims of the present invention is precisely to satisfy such a need.

This need is satisfied by the use of at least one molecule of angiotensinogen or derivatives thereof, notably consisting of des (Angl)AGT or RCL-cleaved AGT, in the manufacture of a drug for use in the treatment of angiogenesis-mediated or dependent pathologies.

Accordingly an object of the present invention is the use of at least one inhibitor of angiogenesis consisting of AGT or derivatives thereof in the manufacture of a drug for treating pathologies or disorders notably angiogenesis-dependent or mediated pathologies generally characterised by excessive angiogenesis.

According to the present inventors, an advantage of the use of the present invention is notably to provide a more appropriate treatment of angiogenesis-mediated diseases with less or no side effects compared to the current treatments. The use of des(Angl)AGT for example, will allow to deliver an antiangiogenic compound devoid of any other known physiological or pharmacological effect. No side effect could be expected from the proposed therapeutic agent.

### Summary of the other objects of the present invention

The present invention also relates to a cell transfected with at least one expression vector wherein said expression vector hosts a recombinant nucleic acid encoding an AGT or one of its derivatives for use in therapy of angiogenesis-mediated pathologies.

The present invention further relates to an expression vector for use in said cell.

The present invention further relates to a method of treating an individual in need of being treated for an angiogenesis-dependent or mediated disease.

Another object of the present invention is a method of treating pathologies or disorders characterised by excessive angiogenesis using AGT or its derivatives molecules comprising des(Ang I)AGT and RCL-cleaved AGT.

### DETAILED DESCRIPTION OF THE INVENTION

The following abbreviations are used in the present disclosure : AGT, angiotensinogen; des(Angl)AGT, des(Angl)angiotensinogen; ATIII, antithrombin III; PEDF, pigment epithelium-derived factor; pV8AGT, protease V8 cleaved AGT; RCL, reactive center loop; HUVECs, human umbilical vein endothelial cells; HMVEC-L, human microvascular endothelial cells from lung; AoSMC, aortic smooth muscle cells; VEGF, vascular endothelial growth factor; bFGF, basic fibroblast growth factor

The present invention relates to the use of at least one molecule of angiotensinogen and derivatives thereof in particular consisting of des(Angl)AGT or RCL-cleaved AGT in the manufacture of a drug for use in the treatment of angiogenesis-mediated or dependent pathologies.

To the best knowledge of the present inventors, AGT or derivatives thereof have never been used in the manufacture of drugs for the treatment of the above mentioned pathologies.

The present invention will now be described in more details by means of the following examples with reference to the attached drawings in which

Figure **1** shows a schematic representation of AGT and its cleaved derivatives. A. AGT comprises 452 amino acids (aa). B. AGT is cleaved by renin to produce the decapeptide angiotensin I and the des(Angl)AGT of 442 aa. C. The protease V8 cleaves a 40 amino acid peptide which remains bound to AGT.

Figure **2** illustrates the anti-angiogenic effect of full length (native)AGT and its cleaved forms des-Angl)AGT and RCL-cleaved AGT in the CAM assay.

A. Normal vascularization of the CAM at day 9 of chick development.

B. AGT inhibited the continuous formation of new small blood vessels of the CAM (asterisks). Note that pre-existing medium- and large sized vessels are not affected (arrows) . Similar anti-angiogenic effects were observed with des(Ang1) AGT(C) and PV8-AGT(D). Bar: 2.5 mm.

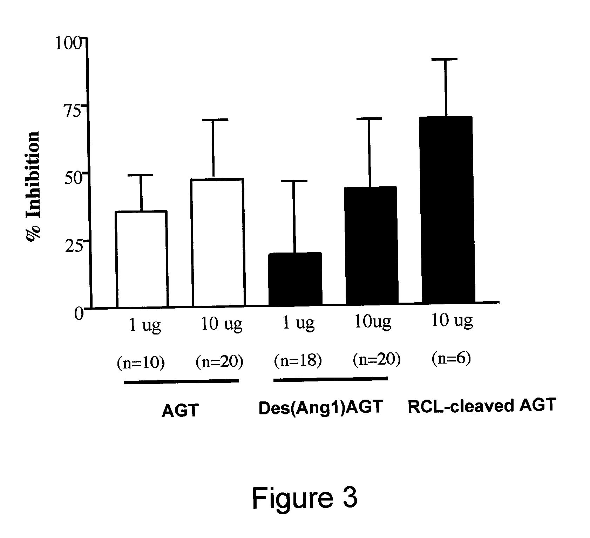

Figure **3** represents the inhibition of the density of vessels induced by AGT and its derivatives des(Ang **I**)AGT and RCL-cleaved AGT.

Percentage of inhibition was evaluated by comparison between the treated area and the non-treated area (0% inhibition) of the same CAM. * p ≤ 0,05; **p<0,01.

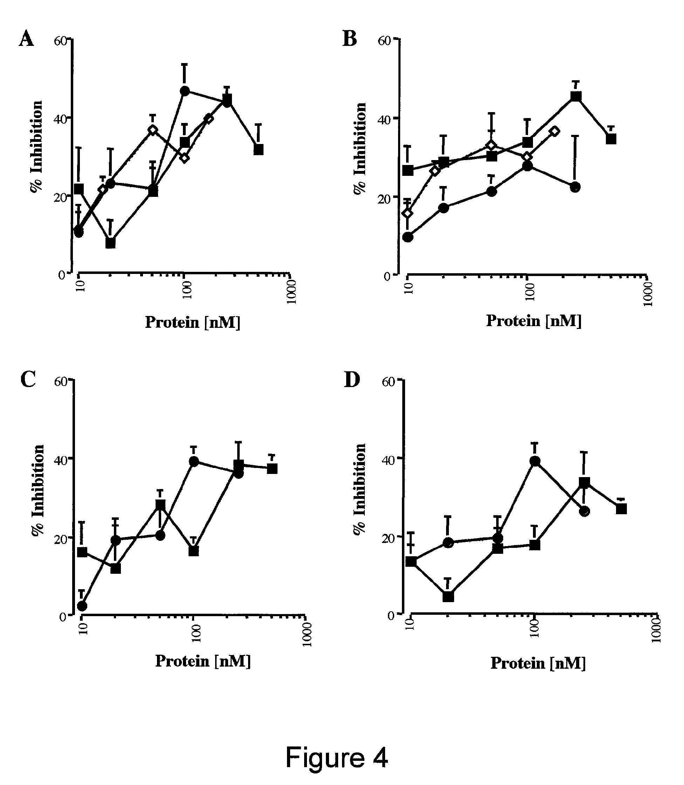

Figure **4** shows the inhibition of endothelial cells proliferation induced by AGT and its derivatives des(Ang I)AGT and RCL-cleaved AGT.

Various concentrations of AGT (■), des(Ang1)AGT (●) and RCL-cleaved AGT(◇) were incubated with HUVEC treated with VEGF (A) or FGF(B), and HMVEC-L treated with VEGF(C) or FGF(D). Results are expressed as percentage of incorporation by comparing to the control in the absence of inhibitors (0% of inhibition). The error bars show the means of triplicate +/-S.D.

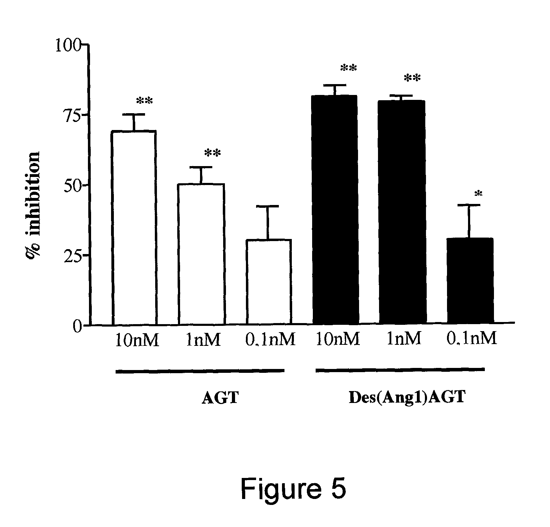

Figure 5 illustrates the inhibition of HUVEC migration by AGT and its derivative des(Ang I)AGT.

Statistical significance by comparison to control groups (0% inhibition). n = 3 in all groups. * p ≤ 0,05 , ** p ≤ 0,01.

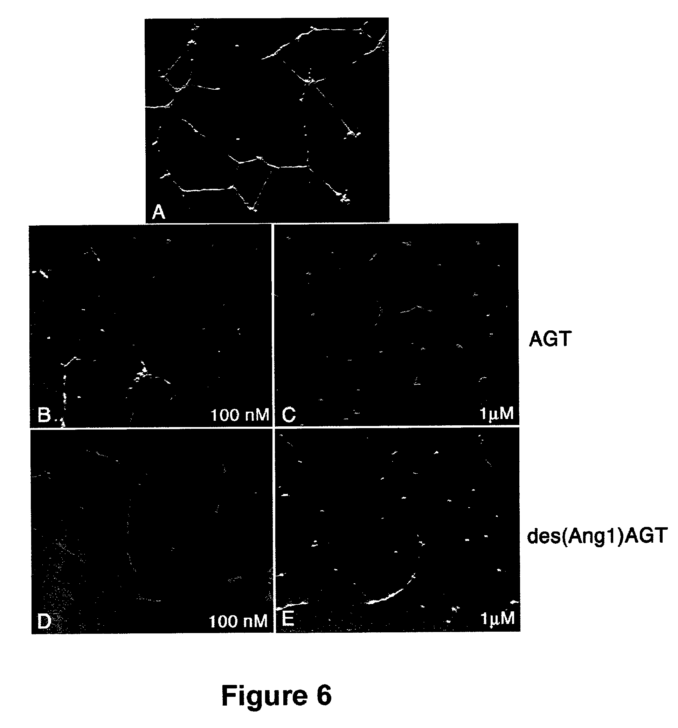

Figure 6 represents the inhibition of *in vitro* capillary tube formation on Matrigel induced by AGT and/or des(Ang I)AGT.

A. Control: HUVEC in complete CBM-2 supplemented with FCS and growth factors (material and methods) showing capillary tubular networks. AGT (B and C) and/OR DES(Ang1) AGT (D and E) showing in a dose-dependent fashion (100nM and 1µM) the inhibition of capillar tube formation. Photographs were taken 24 hours after addition of AGT to the culture medium. Magnification, x30.

### Materials & Methods of the examples Proteins, enzymes and antibodies

Human recombinant AGT was produced in CHO-cells supernatants and was purified to homogeneity, as previously reported by Celerier. Human ATIII and porcine pancreatic elastase (PPE) were obtained from Calbiochem (Bachem, France). The RCL-cleaved ATIII was prepared, using PPE, as previously described by O'Reilly et al., 1999. Purified human recombinant renin was provided by Hoffmann La Roche (Basel, Switzerland) and was used for the generation of des(Ang I)AGT from AGT.

Angiotensinogen anti-peptide antibodies used were Ang I antibody (N-1345) and a C-terminal antibody (C-1350) previously described. A specific des(Ang I)angiotensinogen antibody (D-854) was specifically raised against the first 8 amino-acids residues of the renin product using the synthetic peptide NH₂-Val¹¹-Ile-His-Asn-Glu-Ser-Thr-Cys¹⁸-COOH. The specificity of the reaction against des(Angl)AGT was assessed by pre-immune serum from the same rabbit and its unability to recognize up to 1 µg of AGT.

### Preparation of des(Angl)AGT and proteinase V8 cleaved AGT (RCL-cleaved AGT)

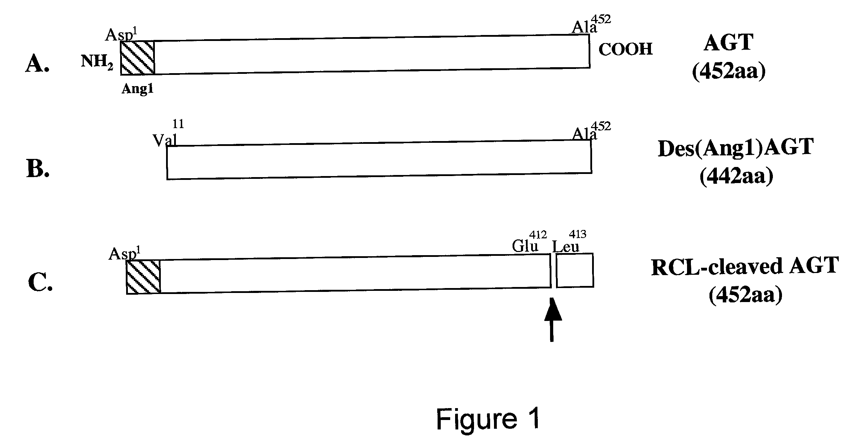

Purified recombinant AGT was extensively hydrolyzed with 1.25 nM purified recombinant human renin in 100 mM citrate/Na₂HPO₄ buffer pH (5.7). The renin reaction was finished by addition of 1 µM pepstatin and Ang I was removed by extensive dialysis against 20 mM Tris-HCI (pH 8.0) buffer containing 150 mM NaCI (buffer A). It was verified that all Angl had been hydrolyzed from AGT and then removed from the des(Angl)AGT preparation. The preparation of des(Angl)AGT obtained was unable to generate Ang I by measuring Angl after extensive cleavage by homologous renin and was however, recognized by a human AGT polyclonal antibody previously described which recognizes as well des(Ang I)AGT and AGT. To further demonstrate that the renin product has lost the Ang I part, a set of anti-peptide antibodies was used. As shown in Fig. 1, whereas AGT possesses the Angl epitope (N-1345) and the intact C-terminal sequence for the mature protein (C-1350), des(Angl)AGT lost the Angl part and acquired a new epitope specific for des(Angl)AGT, now of 442 aa.

Proteinase V8 cleaved AGT (RCL-cleaved AGT) is produced by means of proteinase which cleaves a 40 aa peptide which remains bound to AGT.

### Chick Embryo Chorioallantoic Membrane (CAM) assay

On day 3 of development, fertilized chicken embryos were removed from their shells and placed in plastic Petri dishes, as described by Cruz et al. (2000). On day 7 of incubation, a silicone ring (internal diameter: 10 mm) was laid on the advancing edge of the chicken CAM and proteins in solution applied within the ring. All protein samples were filtered throught a 0.22 µm filter membrane (Prospin X) before application to the CAM and extemporaneously diluted in buffer A. It was verified that there was no inhibition of angiogenesis or embryo letality induced by this buffer alone in at least 40 embryos. Each protein was dissolved in 20 µl of buffer and applied inside the ring. Photographs were taken just before application at day 0, and at day 2 after treatment. Centripetal quantification of the first and second order blood vessels was made in randomly selected zone according to a previously described method on an area representing 1/3 of the treated zone. All observations and evaluation of vascularization were made in a rigorous double blind fashion. Data were statistically treated using a nonparametric t test (StatView software). In the same assay, cleaved ATIII, but not intact ATIII, exhibited a marked anti-angiogenic effect, as described by O'Reilly et al.

### Endothelial cell proliferation

Human umbilical vein endothelial cells (HUVECs) and human microvascular endothelial cells from lung (HMVEC-L) obtained from Clonetics (San Diego, CA) and cultured in EBM-2 (Clonetics) supplemented with fetal calf serum (FCS), 1 µg/ml hydrocortisone-21-acetate, VEGF, IGF, bFGF, and EGF, as recommanded by the manufacturer. Cells were used only from passages 3 to 7.
For proliferation experiments, collagen-coated wells (24-well tissue culture plate (Costar)) were seeded with 5,000 (HUVEC) or 10,000 (HMVEC-L) cells. After 24 hours, endothelial cells were starved in EBM-2 medium containing 0.2% FCS (v/v) for 30 hours. Growth factors [VEGF 165 (3 ng/ml) or bFGF (2.5 ng/ml)] and AGT or des(Angl)AGT were added to the wells at the same time. The cells were incubated for 24 h hours with 3H-thymidine (0.5 µCi/well) (Amersham). Individuals wells were washed twice with ice-cold PBS, before trichloroacetic acid (10%) was added for 1 hour at 4°C, and washed twice with 5% trichloroacetic acid. The remaining material was solubilized with 500 µl 200 mM NaOH and radioactivity incorporated measured by liquid scintillation counting.

### Endothelial cell migration

Endothelial cell migration assays were performed in modified Boyden chambers (Costar), with the upper chamber containing filters of 8.0 µm pore size. The lower chamber was coated with rat tail collagen (0.2 µg/ml). HUVECs were seeded in the upper chamber at a density of 50,000 cells per well (in 200 µl of migration medium (EBM-2/0,1% BSA). Migration was induced by addition of the migration buffer containing 30 ng/ml VEGF (600 µl) in the lower part. AGT and des(Ang I)AGT were added at the indicated doses to the migration medium at the same time as VEGF. HUVECs migration was allowed to performed for 6 hours at 37°C. Cells that remained on the upper side of the filter were removed mechanically. The filters were fixed with 3,7% paraformaldehyde for 1/2 hour. The cells were then stained until crystal violet. The filters were than mounted on glass slides and the number of cells that has migrated to the lower surface were counted in three randomly selected area per filter (x25 magnification). Each assay was done in triplicates.

### Capillary-like tube formation

For capillary-like tube formation assays, Matrigel (Becton Dickinson) was applied into a 24-well tissue culture plate (400 µl per well). After polymerization of the Matrigel (containing or not 1 µM AGT or des(Angl)AGT), HUVECs were seeded (10,000 cells per well) in the culture medium with or without AGT or des(Angl)AGT. After 24h at 37°C the medium was aspirated and cells were fixed in PBS containing 4% (w/v) formaldehyde.

The present invention will now be described in its preferred embodiments and other advantages.

Preferably, in the use according to the present invention, use is made of at least one derivative of AGT which is its enzymatic derivative, in particular via renin for des(Ang I)AGT or via the staphylococcus V8 protease for RCL-cleaved AGT (reactive center loop-cleaved AGT).

More preferably, the present invention relates to the use wherein AGT consists in human recombinant AGT and derivatives via enzymatic cleavage thereof.

Preferably, the use of the present invention concerns pathologies which are selected in the group consisting of pathologies which include, but are not limited to, angiogenesis-dependent cancer including for example, solid tumors and metastases, benign tumors for example hemangiomas, rheumatoid arthritis, psoriasis, for example ischemic retinopathies such as retinopathy of prematurity, proliferative diabetic retinopathy and age-related macular degeneration. They are also useful in the treatment of diseases that have a marked angiogenesis as glioblastomas. These brain tumors expand by intensive neoangiogenesis. AGT and its related compounds could be useful, as they are produced in normal glial cells.

More preferably, the present invention relates to the use according to the present invention wherein the pathology is brain pathology glioblastomas and oligodendrogliomas.

Another preferred embodiment of the present invention is the use of a compound comprising a recombinant nucleic acid encoding an AGT or one of its derivatives inserted within an expression vector wherein said expression result in production of an anti-angiogenic protein. In another preferred embodiment, it relates to an association of the compounds according to the present invention in combination with other anticancer therapy: conventional cytotoxic chemotherapy, radiotherapy, vaccine or immune therapy, delivery of tumor suppressor genes.

More preferably, the present invention relates to the use according to the inventors in combination with the use of another compound selected in the group consisting of anti-angiogenic, anti-cancerous, anti-mitotic drugs or agents destabilizing the vascular wall, or molecules targeting the components of the extra-cellular matrix.

Preferably, in the use according to the present invention the treatment is conducted by local or systemic route.

More preferably, in the use of the present invention, the mode of administration is intravenously, intramuscular, intrathecal, intradermal, intraperitoneal, subcutaneous, intrapleural, intrauterine, rectal, vaginal, topical, intratumor, transdermal or transmucosal.

Another object of the present invention is a cell transfected with at least one expression vector wherein said expression vector comprises a recombinant nucleic acid encoding an AGT or one of its derivatives for use in therapy of angiogenesis-mediated pathologies.

Preferably, the cell according to the present invention is selected in the group consisting of glial cells, endothelial cells, hemopoïetic stem cell precursors (hemangioblast) and liver cells.

A further object of the present invention is an expression vector for use in a cell as described above.

As AGT and its derivatives have anti-angiogenic effects in vitro, these properties can be reproduced by *in vivo* cell targeting of recombinant vectors. These vectors include adenovirus for transient expression and retro-virus for stable expression of AGT or its derivatives in targeted cells. The primary target of such viral constructs are endothelial cells which appear, from experiments reported herein by the inventors, the prime site where AGT and its derivatives exert their anti-angiogenic effects. This strategy of therapeutic use will benefit of the constant progress of this technology.

Another object of the present invention is a method of treating pathologies or disorders characterised by excessive angiogenesis using AGT or its derivatives molecules comprising des(Ang I)AGT and RCL-cleaved AGT.

Preferably, such a method makes use of a cell according to the invention as described above.

More preferably, such a cell is administrated *ex vivo* or *in vivo,* by transient or stable transfection of DNA constructs encoding a complete or partial AGT protein.

A still further object of the present invention is a method which comprises injection of DNA encoding at least one anti-angiogenic molecule selected from the group consisting of AGT and derivatives thereof comprising des(Ang I)AGT and RCL-cleaved AGT.

Such a gene therapy can be employed according to the present invention so as to express and deliver *in vivo* the DNA encoding the full length anti-angiogenic AGT protein, or notably one of its two other derivatives as described above, which also exhibit an anti-angiogenic activity, to a pathological angiogenesis-dependant area.

Various types of pathologies associated with an excessive angiogenesis can be treated by virtue of the new property of the molecules described in the present invention (AGT or its derivatives). In particular, it can be applied for the treatment of retinopathies which affect a substantial number of patients. Since the development of a neovascularization is a crucial step for tumoral growth, such molecules can represent an obvious complementary treatment in addition to classical anti-cancerous drugs used in tumor treatment. Same remark for other pathologies such as retinopathies (ischemic retinopathy of the premature, proliferative diabetic retinopathy), age-related macula degeneration, infantile hemangiomas disorders of female reproduction associated with angiogenesis dysfunction, arthritis, psoriasis, duodenal ulcer.

Other embodiments of the present invention relate more particularly to
- a method of treating diseases and processes that are mediated by angiogenesis comprising AGT and its derivatives des(Ang I)AGT and RCL-cleaved AGT. The angiogenesis inhibiting proteins of the present invention are useful in the treatment of diseases characterized by an excessive or abnormal stimulation of angiogenesis. These pathologies include, but are not limited to, angiogenesis-dependent cancer including for example, solid tumors and metastases, benign tumors for example hemangiomas, rheumatoid arthritis, psoriasis, for example ischemic retinopathies such as retinopathy of prematurity, proliferative diabetic retinopathy and age-related macular degeneration.
- a method of inhibiting angiogenesis using AGT and its derivatives des(Ang I)AGT and RCL-cleaved AGT by a process comprising recombinantly producing these molecules in a recombinant expression system, and isolating the recombinantly produced proteins.
- a method of inhibiting angiogenesis using AGT and its derivatives des(Ang I)AGT and RCL-cleaved AGT wherein a route of administration is intravenously, intramuscular, intrathecal, intradermal, intraperitoneal, subcutaneous, intrapleural, intrauterine, rectal, vaginal, topical, intratumor, transdermal or transmucosal.
- a method of delivering the anti-angiogenic proteins of the present invention (AGT and its derivatives des(Ang I)AGT or RCL-cleaved AGT) using gene therapy strategies comprising injection of DNA.
- diagnostic methods and kit for detection or measurement of a anti-angiogenic protein by use of detection means specific for AGT, des(Ang I)AGT and RCL-cleaved AGT.
- Such methods or kits include: the use of antibodies to the molecule of AGT or its derivatives; the use of peptides whose sequence is derived from that of AGT; the use of chemically modified peptides; all these compounds being designed for the measurement, stimulation or inhibition of the anti-angiogenic properties of AGT or its derivatives.

### EXAMPLES

### EXAMPLE 1

### Full length (Native) AGT and des(Ang I)AGT and RCL-cleaved AGT are anti-angiogenic in the CAM

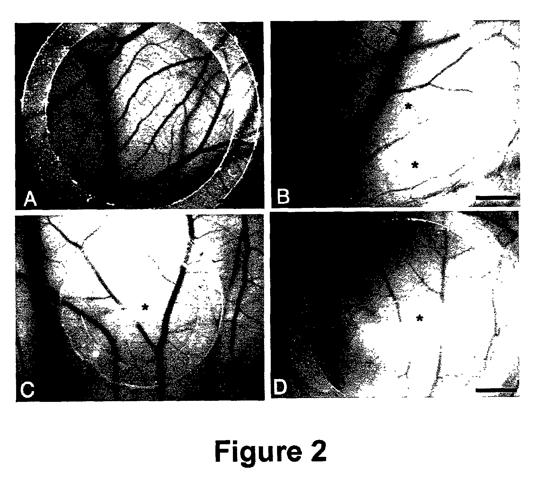

Human AGT and its cleaved derivatives were applied onto the CAM at day 7 of incubation, at a time of intensive angiogenesis. The three molecules were able to inhibit spontaneous angiogenesis (Fig. 2B through 2D). In order to quantify this effect, first and second order microvessels were counted in a blind fashion in zones representing around 1/3 of the internal surface of the ring (diameter: 1cm). The anti-angiogenic effect of AGT, des(Angl)AGT and RCL-cleaved AGT was due to a reduction of the first and second order vessels but larger vessels were not affected. Treatment with 1 µg of both AGT or des(Angl)AGT led to a slight but significant inhibition of the density of vessels (Figure 3). Treatment with 10 µg led to a more pronounced inhibition of spontaneous angiogenesis for the three molecules studied. The density of first and second order vessels in AGT and des(Angl)AGT-treated CAM was reduced to less than 50% of non-treated zones of the same embryo (p<0.01). RCL-cleaved AGT at 10 µg showed a reduction of 65%.There was no significant difference of the anti-angiogenic effect of AGT and des(Angl)AGT in this assay.

According to the present inventors, it is the first time that it is disclosed the anti-angiogenic effect of molecules such as AGT, des(Angl)AGT and RCL-cleaved AGT in CAM assay.

### EXAMPLE 2

### Antiproliferative effect of AGT and des(Ang I)AGT on endothelial cells

AGT, des(Angl)AGT and RCL-cleaved AGT inhibit endothelial cell (HUVEC and HMVEC 3-L) proliferation induced by VEGF in a dose-dependent fashion (Fig 4, A and C). Both molecules exhibited a similar effect with a half maximal inhibition (EC_{50%}) at approximately 100-200 nM. Similar results, with an EC_{50%} of around 100 nM, were obtained when endothelial cells were pre-treated with FGF-2 (Fig. 4, B and D).
Non-endothelial cells (AoSMC or human keartinocytes) were not responsive to the inhibitory effect of AGT and des(Angl)AGT even using a 10-times higher concentration inhibitors (data not shown).

According to the present inventors, it is the first time that it is disclosed the antiproliferative effect of molecules such as AGT, des(Angl)AGT and RCL-cleaved AGT on endothelial cells.

### EXAMPLE 3:

It has been shown by the inventors that AGT and its derivatives exert strong effects not only on proliferation of endothelial cells but also on another essential property of these cells: migration.

Increasing concentrations of AGT or its derivative Des(Angl)AGT inhibit the migration of HUVECs through a filter (pore size 20µm) separating two compartments in a cell culture dish. Cells seeded in one compartment (origin) are attracted in the other compartment (target) by FGF-2 OR VEGF (see figure 5) and this chemotropic effect is antagonized by AGT and its derivatives. This inhibition is measured by the ratio between the number of HUVECs found in the target compartment in the presence of AGT (n% of inhibition) compared to that in the absence of AGT (0% of inhibition).

This migratory behavior of endothelial cells is essential to angiogenesis and the power to block this migration is part of the anti-angiogenic strategy of using AGT and its derivatives.

### EXAMPLE 4

### Inhibition of capillary-like structure on Matrigel by AGT and des(Ang I)AGT

The ability to form capillary-like structures in Matrigel is another specific property of endothelial cells and another important step of angiogenesis. HUVECs seeded on Matrigel rapidly form structures (within six hours, Fig 6A). A network of capillary-like extensions with numerous intercellular contacts. Addition of AGT or des(Ang I)AGT (100 nM and 1 µM within the Matrigel produced an obvious reduction in length of the capillary-like structures and number of their junctions in dose-dependent fashion (Fig 6, B through E).

According to the present inventors, it is the first time that it is disclosed the Inhibition of capillary-like structure of molecules such as AGT and des(Angl)AGT on Matrigel.

The present inventors have herein demonstrated a new property of three AGT species: AGT, des(Angl)AGT and RCL-cleaved AGT. All exhibited similar effects in well established models of angiogenesis and endothelial cell proliferation and migration. Moreover, they demonstrated that the anti-angiogenic activity of AGT and its derivatives is independent of its serpin inhibitory activity. Indeed, neither the release of Angl nor the cleavage of the RCL affected the anti-angiogenic property of AGT.

Such property allows the manufacture of new drugs for use in the treatment of pathologies characterized by an excessive angiogenesis

### REFERENCES

1. Barrett, J. D., Eggena, P., Hidaka, H., and Sambhi, M. P. (1979) *In vitro* Inhibition of Renin by Human Des-Angiotensin I Renin Substrate. Journal of Clinical Endocrinology and Metabolism 8, 96-100.
2. Becerra, S. P., Sagasti, A., Spinella, P., and Notario, V. (1995) Pigment epithelium-derived factor behaves like a noninhibitory serpin. Neurotrophic activity does not require the serpin reactive loop. J Biol Chem 270, 25992-9.
3. Becerra, S. P. (1997) Structure-function studies on PEDF. A noninhibitory serpin with neurotrophic activity. Adv Exp Med Biol 425, 223-37.
4. Catanzaro, D. F., Sun, J., Gilbert, M. T., Yan, Y., Black, T., Sigmund, C., and Gross, K. W. (1994) A Pit-1 binding site in the human renin gene promoter stimulates activity in pituitary, placental and juxtaglomerular cells. Kidney Int 46, 1513-5.
5. Celerier, J., Schmid, G., Le Caer, J. P., Gimenez-Roqueplo, A. P., Bur, D., Friedlein, A., Langen, H., Corvol, P., and Jeunemaitre, X. (2000) Characterization of a human angiotensinogen cleaved in its reactive center loop by a proteolytic activity from Chinese hamster ovary cells. J Biol Chem 275, 10648-54.
6. Christiansen, M., Jaliashvili, I., Overgaard, M. T., Ensinger, C., Obrist, P., and Oxvig, C. (2000) Quantification and characterization of pregnancy-associated complexes of angiotensinogen and the proform of eosinophil major basic protein in serum and amniotic fluid. Clin Chem 46, 1099-105.
7. Dawson, D. W., Volpert, O. V., Gillis, P., Crawford, S. E., Xu, H., Benedict, W., and Bouck, N. P. (1999) Pigment epithelium-derived factor: a potent inhibitor of angiogenesis. Science 285, 245-8.
8. DeFouw, D. O., Rizzo, V. J., Steinfeld, R., and Feinberg, R. N. (1989) Mapping of the microcirculation in the chick chorioallantoic membrane during normal angiogenesis. Microvasc Res 38, 136-47.
9. Fitzpatrick, P. A., Wong, D. T., Barr, P. J., and Pemberton, P. A. (1996) Functional implications of the modeled structure of maspin. Protein Eng 9, 585-9.
10. Genain, C., Bouhnik, J., Tewksbury, D., Corvol, P., and Ménard, J. (1984) Characterization of plasma and CSF human angiotensinogen and des-angiotensin I angiotensinogen by direct radioimmunoassay. J. Clin. Endocrinol. Metab. 59, 478-484.
11. Gimenez-Roqueplo, A. P., Celerier, J., Schmid, G., Corvol, P., and Jeunemaitre, X. (1998) Role of cysteine residues in human angiotensinogen. Cys232 is required for angiotensinogen-pro major basic protein complex formation. J Biol Chem 273, 34480-7.
12. Gimenez-Roqueplo, A. P., Celerier, J., Lucarelli, G., Corvol, P., and Jeunemaitre, X. (1998) Role of N-glycosylation in human angiotensinogen. J Biol Chem 273, 21232-8.
13. Gutkowska, J., Corvol, P., Figueiredo, A. F., Inagami, T., Bouhnik, J., and Genest, J. (1984) Kinetic studies of rat renin and tonin on purified rat angiotensinogen. Can J Biochem Cell Biol 62, 137-42.
14. Ishiguro, K., Kojima, T., Kadomatsu, K., Nakayama, Y., Takagi, A., Suzuki, M., Takeda, N., Ito, M., Yamamoto, K., Matsushita, T., Kusugami, K., Muramatsu, T., and Saito, H. (2000) Complete antithrombin deficiency in mice results in embryonic lethality [In Process Citation]. J Clin Invest 106, 873-8.
15. Jeunemaitre, X., Soubrier, F., Kotelevtsev, Y., Lifton, R. P., Williams, C. S., Charru, A., Hunt, S. C., Hopkins, P. N., Williams, R. R., Lalouel, J.-M., and Corvol, P. (1992) Molecular basis of human hypertension. Role of angiotensinogen. Cell 71, 169-178.
16. Jeunemaitre, X., Charru, A., Chatellier, A., Dumont, C., Sassano, P., Soubrier, F., Ménard, J., and Corvol, P. (1993) M235T variant of the human angiotensinogen gene in unselected hypertensive patients. J. Hypertens. 11 (suppl 5), S80-S81.
17. Kakinuma, Y., Hama, H., Sugiyama, F., Yagami, K., Goto, K., Murakami, K., and Fukamizu, A. (1998) Impaired blood-brain barrier function in angiotensinogen-deficient mice. Nat Med 4, 1078-80.
18. Larsson, H., Sjoblom, T., Dixelius, J., Ostman, A., Ylinenjarvi, K., Bjork, I., and Claesson-Welsh, L. (2000) Anti-angiogenic effects of latent antithrombin through perturbed cell- matrix interactions and apoptosis of endothelial cells. Cancer Res 60, 6723-9.
19. le Noble, F. A., Kessels-van Wylick, L. C., Hacking, W. J., Slaaf, D. W., oude Egbrink, M. G., and Struijker-Boudier, H. A. (1996) The role of angiotensin II and prostaglandins in arcade formation in a developing microvascular network. J Vasc Res 33, 480-8.
20. Machado, R. D., Santos, R. A., and Andrade, S. P. (2000) Opposing actions of angiotensins on angiogenesis. Life Sci 66, 67-76.
21. Mathews, S., Döbeli, H., Pruschy, M., Bosser, R., D'Arcy, A., Oefner, C., Zulauf, M., Gentz, R., Breu, V., Matile, H., Schlaeger, J., and Fischli, W. (1996) Recombinant human renin produced in different expression systems: biochemical properties and 3D structure. Protein Expression and Purification 7, 81-91.
22. Matsui, T., Tamaya, K., Matsumoto, K., Osajima, Y., Uezono, K., and Kawasaki, T. (1999) Plasma concentrations of angiotensin metabolites in young male normotensive and mild hypertensive subjects. Hypertens Res 22, 273-7.
23. Ménard, J., and Catt, K. J. (1972) Measurement of renin activity, concentration and substrate in rat plasma by radioimmunoassy of angiotensin I. Endocrinology 90, 422-430.
24. Moraga, F., and Janciauskiene, S. (2000) Activation of primary human monocytes by the oxidized form of alpha1- antitrypsin. J Biol Chem 275, 7693-700.
25. Mourey, L., Samama, J. P., Delarue, M., Petitou, M., Choay, J., and Moras, D. (1993) Crystal structure of cleaved bovine antithrombin III at 3.2 A resolution. J Mol Biol 232, 223-41.
26. O'Reilly, M. S., Pirie-Shepherd, S., Lane, W. S., and Folkman, J. (1999) Anti-angiogenic activity of the cleaved conformation of the serpin antithrombin [see comments]. Science 285, 1926-8.
27. Patston, P. A., and Gettins, P. G. (1996) Significance of secondary structure predictions on the reactive center loop region of serpins: a model for the folding of serpins into a metastable state. FEBS Lett 383, 87-92.
28. Pemberton, P. A., Wong, D. T., Gibson, H. L., Kiefer, M. C., Fitzpatrick, P. A., Sager, R., and Barr, P. J. (1995) The tumor suppressor maspin does not undergo the stressed to relaxed transition or inhibit trypsin-like serine proteases. Evidence that maspin is not a protease inhibitory serpin. J Biol Chem 270, 15832-7.
29. Petitclerc, E., Boutaud, A., Prestayko, A., Xu, J., Sado, Y., Ninomiya, Y., Sarras, M. P., Jr., Hudson, B. G., and Brooks, P. C. (2000) New functions for non-collagenous domains of human collagen type IV. Novel integrin ligands inhibiting angiogenesis and tumor growth in vivo. J Biol Chem 275, 8051-61.
30. Poulsen, K., and Jacobsen, J. (1986) Is angiotensinogen a renin inhibitor and not the substrate for renin? J Hypertens 4, 65-9.
31. Rizzo, V., and DeFouw, D. O. (1996) Mast cell activation accelerates the normal rate of angiogenesis in the chick chorioallantoic membrane. Microvasc Res 52, 245-57.
32. Smith, L. E., Shen, W., Perruzzi, C., Soker, S., Kinose, F., Xu, X., Robinson, G., Driver, S., Bischoff, J., Zhang, B., Schaeffer, J. M., and Senger, D. R. (1999) Regulation of vascular endothelial growth factor-dependent retinal neovascularization by insulin-like growth factor-1 receptor. Nat Med 5, 1390-5.
33. Stein, P. E., Tewkesbury, D. A., and Carrell, R. W. (1989) Ovalbumin and angiotensinogen lack serpin S-R conformational change. Biochem J 262, 103-7.
34. Streatfeild-James, R. M., Williamson, D., Pike, R. N., Tewksbury, D., Carrell, R. W., and Coughlin, P. B. (1998) Angiotensinogen cleavage by renin: importance of a structurally constrained N-terminus. FEBS Lett 436, 267-70.
35. Struman, I., Bentzien, F., Lee, H., Mainfroid, V., D'Angelo, G., Goffin, V., Weiner, R. I., and Martial, J. A. (1999) Opposing actions of intact and N-terminal fragments of the human prolactin/growth hormone family members on angiogenesis: an efficient mechanism for the regulation of angiogenesis. Proc Natl Acad Sci U S A 96, 1246-51.
36. Wintroub, B. U., Klickstein, L. B., Dzau, V. J., and Watt, K. W. (1984) Granulocyte-angiotensin system. Identification of angiotensinogen as the plasma protein substrate of leukocyte cathepsin G. Biochemistry 23, 227-32.
37. Xia, W., Lau, Y. K., Hu, M. C., Li, L., Johnston, D. A., Sheng, S., El-Naggar, A., and Hung, M. C. (2000) High tumoral maspin expression is associated with improved survival of patients with oral squamous cell carcinoma. Oncogene 19, 2398-403.
38. Yanai, K., Saito, T., Kakinuma, Y., Kon, Y., Hirota, K., Taniguchi-Yanai, K., Nishijo, N., Shigematsu, Y., Horiguchi, H., Kasuya, Y., Sugiyama, F., Yagami, K., Murakami, K., and Fukamizu, A. (2000) Renin-dependent cardiovascular functions and renin-independent blood- brain barrier functions revealed by renin-deficient mice. J Biol Chem 275, 5-8.
39. Zhang, M., Volpert, O., Shi, Y. H., and Bouck, N. (2000) Maspin is an angiogenesis inhibitor [In Process Citation]. Nat Med 6, 196-9.
40. Zou, Z., Anisowicz, A., Hendrix, M. J., Thor, A., Neveu, M., Sheng, S., Rafidi, K., Seftor, E., and Sager, R. (1994) Maspin, a serpin with tumor-suppressing activity in human mammary epithelial cells [see comments]. Science 263, 526-9.

## Claims

1. Use of at least one molecule of angiotensinogen or derivatives thereof consisting of des(Angl)AGT or RCL-cleaved AGT in the manufacture of a drug for use in the treatment of angiogenesis-mediated or dependent pathologies.

2. Use according to claim 1, wherein AGT derivative is des(Angl)AGT.

3. Use according to claim 1, wherein AGT derivative is RCL-cleaved AGT.

4. Use according to anyone of claims 1 to 3, wherein AGT consists in human recombinant AGT and its derivatives via enzymatic cleavage thereof.

5. Use according to anyone of claims 1 to 4, wherein the pathologies are selected in the group consisting of angiogenesis-dependent cancer including for example, solid tumors and metastases, benign tumors for example hemangiomas, rheumatoid arthritis, psoriasis, for example ischemic retinopathies such as retinopathy of prematurity, proliferative diabetic retinopathy and age-related macular degeneration..

6. Use according to claim 5, wherein the pathology is brain pathology such as glioblastoma and oligodendroglioma.

7. Use of a compound comprising at least one recombinant nucleic acid encoding an AGT or one of its derivatives inserted within an expression vector wherein said expression results in production of an anti-angiogenic protein.

8. Use according to claim 7, wherein the AGT derivatives is des(Angl)AGT.

9. Use according to anyone of the preceding claim, in combination with the use of an other compound selected in the group consisting of anti-angiogenic, anti-cancerous , anti-mitotic compounds or agents destabilizing the vascular wall, or molecules targeting the components of the extra-cellular matrix.

10. Use according to anyone of the preceding claims, wherein the anti-angiogenic treatment is conducted by local or systemic routes.

11. Use according to claim 10, wherein the mode of administration is intravenously, intramuscular, intrathecal, intradermal, intraperitoneal, subcutaneous, intrapleural, intrauterine, rectal, vaginal, topical, intratumor, transdermal or transmucosal.

12. Cell transfected with at least one expression vector wherein said expression vector comprises a recombinant nucleic acid encoding an AGT or one of its derivatives for use in therapy of angiogenesis-mediated pathologies.

13. Cell according to claim 12, wherein the cell is selected in the group consisting of glial cells, blood cells or endothelial cells.

14. Cell according to claim 12, wherein said cells are selected in the group consisting of tumor cells.

15. Expression vector for use in a cell according to anyone of claims 13 and 14.

16. Expression vector according to claim 15, wherein the expression vector hosts a recombinant nucleic acid being des(Angl)AGT.
